# EUROPEAN PATENT APPLICATION

(11) **EP 4 483 923 A1**
(43) Date of publication of application: **01.01.2025**
(21) Application number: 23760406.1
(22) Date of filing: 23.02.2023
(51) Int. Cl.: A61M 5/145, A61M 5/165, A61M 5/36, A61M 5/315

(54) **AIR-FILLED MEDICINAL LIQUID PUMPING APPARATUS AND MEDICINAL LIQUID INJECTION APPARATUS**

(30) Priority: 23.02.2022 KR 20220023323
(71) Applicant: Kim, Yong Hyun, Goyang-si, Gyeonggi-do 10483 (KR)
(72) Inventor: Kim, Yong Hyun, Goyang-si, Gyeonggi-do 10483 (KR)
(74) Representative: Santarelli
(86) International application number: PCT/KR2023/002623
(87) International publication number: WO 2023/163532

(57) **Abstract**

There is provided a technique that includes a chamber configured to form an injection flow path connecting an air-filled internal space with an outside of the chamber. The chamber includes an inner upper end surface defining an upper end of the internal space, and an inner protrusion protruding downward from the inner upper end surface and including a protruding end where a connection hole, which is one end of the injection flow path, is formed; and a plunger configured to form the internal space together with the chamber and be movable in a vertical direction along an inner surface of the chamber to change a volume of the internal space. The plunger includes an upper surface forming an insertion groove recessed downward to allow the inner protrusion to be inserted in the insertion groove. The inner upper end surface and the upper surface are spaced apart from each other.

## Description

### TECHNICAL FIELD

The present disclosure relates to an air-filled medicinal liquid pumping apparatus, a medicinal liquid injection apparatus, and a method of manufacturing the air-filled medicinal liquid pumping apparatus.

### BACKGROUND

There is known a medicinal liquid injection apparatus for injecting a medicinal liquid (e.g., an injection solution) in a liquid state into a patient in order to supply the medicinal liquid to the patient. By using the medicinal liquid injection apparatus, the medicinal liquid in a predetermined storage space passes through a passage (e.g., the internal spaces of a tube and an injection needle) connected to the patient and flows into the body of the patient.

A conventional medicinal liquid injection apparatus includes a chamber that defines the above-described storage space therein, and a plunger that slides along the inner surface of the chamber and changes the volume of the above-described storage space. As the plunger slides in a predetermined pressing direction with respect to the chamber and the volume of the storage space decreases, the medicinal liquid in the storage space moves along the above-described passage and flows into the patient's body. In a state in which the plunger slides as much as possible in the above-described pressing direction with respect to the chamber, the medicinal liquid remains in the passage described above, and the remaining medicinal liquid cannot be injected into the patient's body and may generally be discarded. That is, a portion of the total amount of medicinal liquid in the storage space of the medicinal liquid injection apparatus flows into the patient's body, but the remaining portion remains in the above-described passage and cannot be used.

### SUMMARY

In the case of the above-described conventional medicinal liquid injection apparatus, there is a problem in that a significant amount of medicinal liquid remaining in the above-described passage is wasted. The present disclosure solves the problems of the conventional medicinal liquid injection apparatus.

An aspect of the present disclosure provides a medicinal liquid pumping apparatus. In an representative embodiment, the medicinal liquid pumping apparatus may include: a chamber configured to form an injection flow path connecting an air-filled internal space with an outside of the chamber, wherein the chamber includes an inner upper end surface defining an upper end of the internal space, and an inner protrusion protruding downward from the inner upper end surface and including a protruding end where a connection hole, which is one end of the injection flow path, is formed; and a plunger configured to form the internal space together with the chamber and be movable in a vertical direction along an inner surface of the chamber to change a volume of the internal space, wherein the plunger includes an upper surface forming an insertion groove recessed downward to allow the inner protrusion to be inserted in the insertion groove. The inner upper end surface and the upper surface are spaced apart from each other.

In an embodiment, an upper end of the plunger may be located below a lower end of the inner protrusion.

In an embodiment, the chamber may have an air collection groove recessed upward along a circumference of the inner protrusion and forming a portion of the internal space.

In an embodiment, the plunger may include an insertion portion that protrudes upward along a circumference of the insertion groove and is configured to be engaged with the air collection groove when the plunger moves upward.

In an embodiment, air filling the internal space may be sterilized air.

In an embodiment, the medicinal liquid pumping apparatus may be manufactured in a state in which the internal space is filled with air.

In an embodiment, the medicinal liquid pumping apparatus may further include a pressing operation part configured to press the plunger so as to move the plunger upward.

Another aspect of the present disclosure provides a medicinal liquid injection apparatus. In an representative embodiment, the medicinal liquid injection apparatus may include: the air-filled medicinal liquid pumping apparatus; an injection-part-fixing device configured such that an injection part configured to be inserted into a patient's skin for injecting a medicinal liquid is coupled to the injection-part-fixing device, wherein the injection-part-fixing device is configured to form a guide flow path configured to guide a flow of the medicinal liquid to the injection part; an air filter coupled to the injection-part-fixing device and connected to an upstream end of the guide flow path to form a filter flow path configured to guide the flow of the medicinal liquid into the guide flow path, wherein the air filter is configured to filter air in the medicinal liquid and discharge the air to the outside of the chamber; and a connection tube part configured to connect an upstream end of the filter flow path of the air filter with a downstream end of the injection flow path of the air-filled medicinal liquid pumping apparatus, wherein the connection tube part is configured to form a connection flow path configured to guide the flow of the medicinal liquid.

In an embodiment, a volume of the internal space located above an imaginary horizontal line that is in contact with the protruding end of the inner protrusion may be greater than or equal to a sum of a volume of the injection flow path and a volume of the connection flow path.

In an embodiment, a sum of a volume of the filter flow path and a volume of the guide flow path may be smaller than the sum of the volume of the injection flow path and the volume of the connection flow path.

In an embodiment, the injection-part-fixing device includes: a fixed body configured to form the guide flow path; and a support leg configured to support the fixed body with respect to a patient's skin.

According to the embodiments of the present disclosure, through a structure of a medicinal liquid pumping apparatus that allows air to push out the medicinal liquid remaining in a connection tube, the amount of medicinal liquid remaining in the connection tube after use of the medicinal liquid pumping apparatus can be significantly reduced.

According to an embodiment of the present disclosure, by providing the air-filled medicinal liquid pumping apparatus in the state in which the internal space is filled with air, even when the medicinal liquid is injected into the internal space, air capable of pushing the medicinal liquid remaining in the connection tube after use of the medicinal liquid injection apparatus can be always present in the internal space.

According to an embodiment of the present disclosure, even if the plunger slides upward relative to the chamber, the state in which air is trapped in the air collection groove of the chamber is maintained. Thus, after the medicinal liquid flows out of the internal space of the medicinal liquid pumping apparatus, the trapped air can be induced to push the medicinal liquid remaining in the connection tube while flowing out from the internal space.

According to an embodiment of the present disclosure, the air in the internal space can be induced to be pressed by the plunger just before the plunger slides upward with respect to the chamber as much as possible.

According to the medicinal liquid injection apparatus according to an embodiment of the present disclosure, the medicinal liquid within the injection flow path of the chamber and the connection flow path of the connection tube part can be pushed out as much as possible by the air trapped around the inner protrusion inside the chamber, and the air pushing out the medicinal liquid can be filtered by the air filter before entering a patient's body.

According to the medicinal liquid injection apparatus according to an embodiment of the present disclosure, the volume of the internal space located above an imaginary horizontal line that is in contact with the protruding end of the inner protrusion is set to be larger than or equal to the sum of the volume of the injection flow path and the volume of the connection flow path. Thus, all of the medicinal liquid in the injection flow path and the connection flow path, which are flow paths upstream of the air filter, can be pushed out with the air trapped around the inner protrusion inside the chamber.

### BRIEF DESCRIPTION OF THE DRAWINGS

FIG. 1 is a conceptual view illustrating the entire system of a medicinal liquid injection set 1 and 2.
FIG. 2 is an enlarged cross-sectional view of the air-filled medicinal liquid pumping apparatus of FIG. 1.
FIG. 3 is an enlarged cross-sectional view illustrating the state in which the air-filled medicinal liquid pumping apparatus of FIG. 2 is filled with a medicinal liquid.
FIG. 4 is an enlarged cross-sectional view illustrating the state in which the plunger of FIG. 3 moves upward.
FIG. 5 is a flowchart illustrating an operation method of the air-filled medicinal liquid pumping apparatus.

### DETAILED DESCRIPTION

Embodiments of the present disclosure are exemplified for describing the technical contents of the present disclosure. The scope of rights according to the present disclosure is not limited to the embodiments presented below or the specific description of these embodiments.

All technical terms and scientific terms used in the present disclosure have meanings that are commonly understood by a person ordinarily skilled in the art to which the present disclosure belongs unless otherwise defined. All of the terms used in the present disclosure are selected for the purpose of describing the present disclosure more clearly, and are not selected to limit the scope of rights according to the present disclosure.

As used in the present disclosure, expressions such as "comprising," "including," "having," and the like are to be understood as open-ended terms having the possibility of encompassing other embodiments, unless otherwise mentioned in the phrase or sentence including such expressions.

Singular expressions that are described in the present disclosure may encompass plural expressions unless otherwise stated, which also applies to the singular expressions recited in the claims.

As used in the present disclosure, expressions such as "first," "second," and the like are used to distinguish a plurality of elements from each other, and are not intended to limit an order or importance of the corresponding elements.

In the present disclosure, the description that one element is "connected" or "coupled" to another element is to be understood to indicate that said one element may be directly connected or coupled to said another element, and is to be further understood that a new element may be interposed between the one element and the other element.

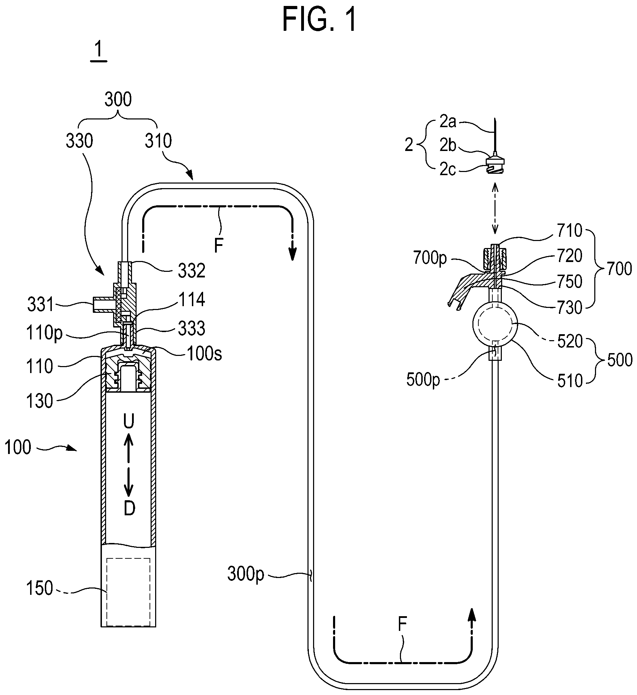

As used in the present disclosure, "upstream" and "downstream" are defined based on the direction in which a medicinal liquid flows when the air-filled medicinal liquid pumping apparatus 100 presses the medicinal liquid. Specifically, the direction of the arrow F in FIG. 1 is defined as the downstream direction, and the opposite direction to the downstream direction is defined as the upstream direction.

Directional terms used in the present disclosure, such as "upward" and "upper," are based on the direction in which the plunger slides relative to the chamber to reduce the volume of the internal space of the medicinal liquid pumping apparatus in the accompanying drawings, and the directional terms, such as "downward" and "lower," mean the opposite direction. In the drawings, the upward direction U and the downward direction D are indicated.

Hereinafter, embodiments of the present disclosure will be described with reference to the accompanying drawings. In the accompanying drawings, identical or corresponding components may be assigned the same reference numerals. In addition, in the following description of the embodiments, redundant descriptions for the same or corresponding elements may be omitted. However, even if descriptions of components are omitted, it is not intended that such components are not included in any embodiment.

FIG. 1 is a conceptual view illustrating the entire system of a medicinal liquid injection set 1 and 2. Referring to FIG. 1, a medicinal liquid injection set 1 and 2 according to an embodiment of the present disclosure includes a medicinal liquid injection apparatus 1 and an injection part 2 configured to be coupled to the medicinal liquid injection apparatus 1. For example, after filling a priming liquid in a connection tube part 300, which will be described later, in a state in which an end cap (not illustrated) having a hydrophobic filter is coupled to an injection-part-fixing device 700, which will be described later, the end cap may be separated from the injection-part-fixing device 700, and the injection part 2 may be connected to the injection-part-fixing device 700.

The injection part 2 is configured to be inserted into a patient's skin and inject a medicinal liquid. The injection part 2 may include an injection needle 2a, a catheter, or the like. The injection part 2 according to an embodiment includes an injection needle 2a and an injection needle support 2b that supports the injection needle 2a. The injection part 2 includes a corresponding coupling portion 2c configured to be coupled to an injection-part-coupling portion 710 of the injection-part-fixing device 700. The injection part 2 may be configured by sequentially connecting the injection needle 2a, the injection needle support 2b, and the corresponding coupling portion 2c.

The medicinal liquid injection apparatus 1 includes an air-filled medicinal liquid pumping apparatus 100. The medicinal liquid injection apparatus 1 includes a connection tube part 300 connected to an injection flow path 110p, which will be described later, of the air-filled medicinal liquid pumping apparatus 100. The medicinal liquid injection apparatus 1 may further include an air filter 500 coupled to a downstream end of the connection tube part 300. The medicinal liquid injection apparatus may further include an injection-part-fixing device 700 coupled to a downstream end of the air filter 500.

The medicinal liquid flows into a patient's body in response to pressing in the air-filled medicinal liquid pumping apparatus 100. The medicinal liquid is flowable into the patient's body sequentially from the air-filled medicinal liquid pumping apparatus 100 through the connection tube part 300, the air filter 500, the injection-part-fixing device 700, and the injection part 2.

The air-filled medicinal liquid pumping apparatus 100 includes a chamber 110 configured to accommodate a medicinal liquid. The chamber 110 forms an internal space 100s together with the plunger 130. A medicinal liquid may be stored in the internal space 100s. The chamber 110 may include a coupling portion 114 to which the connection tube part 300 is coupled. The coupling portion 114 may be coupled to a port connection portion 333 of a port part 330. The coupling portion 114 may be provided on an upper side of the chamber 110. The coupling portion 114 may include a tubular member extending in the vertical direction. The coupling portion 114 may form an injection flow path 110p.

The air-filled medicinal liquid pumping apparatus 100 is configured to press the medicinal liquid. The air-filled medicinal liquid pumping apparatus 100 includes a plunger 130 that presses the medicinal liquid inside the chamber 110. The plunger 130 may press the medicinal liquid inside the chamber 110 by moving upward. When the medicinal liquid is being filled into the chamber 110, the plunger 130 moves downward.

The connection tube part 300 forms a connection flow path 300p configured to guide the flow of the medicinal liquid. The connection tube part 300 may guide the movement of the medicinal liquid from the air-filled medicinal liquid pumping apparatus 100 to the air filter 500. The connection tube part 300 is configured to allow the medicinal liquid flowing out of the air-filled medicinal liquid pumping apparatus 100 to flow therethrough in response to pressing in the air-filled medicinal liquid pumping apparatus 100. The connection flow path 300p interconnects a downstream end of an injection flow path 110p of the air-filled medicinal liquid pumping apparatus 100 and an upstream end of a filter flow path 500p of the air filter 500.

The connection tube part 300 includes a connection tube 310 that forms at least a portion of the connection flow path 300p. The connection tube part 300 may further include a port part 330 that forms a portion of the connection flow path 300p. For example, the port part 330 may form a partial flow path of an upstream portion of the connection flow path 300p, and the connection tube 310 may form the remaining portion of the connection flow path 300p.

The port part 330 is configured to fill a medicinal liquid into the medicinal liquid pumping apparatus 100. The medicinal liquid may flow into the medicinal liquid pumping apparatus 100 through the port part 330 from the outside. The medicinal liquid is flowable into the internal space 100s of the medicinal liquid pumping apparatus 100 through the port part 330. The port part 330 may include a port inlet 331 configured to allow a medicinal liquid to flow in from the outside. The medicinal liquid is filled into the internal space 100s of the medicinal liquid pumping apparatus 100 through the port inlet 331, and the plunger 130 moves downward.

The port part 330 may be connected to the chamber 110 and the connection tube 310. The port part 330 is disposed between the chamber 110 and the connection tube 310 to interconnect the chamber 110 and the connection tube 310. The port part 330 may include a port extension 332 connected to an upstream end of a first connection portion 310 of the connection tube 310, which will be described later. The medicinal liquid in the internal space 100s of the chamber 110 may flow into the connection tube 310 through the port extension 332 of the port part 330.

The port part 330 may include a port connection portion 333 connected to the chamber 110. The port connection portion 333 may be coupled to the coupling portion 114 of the chamber 110. The port connection portion 333 may be configured to extend vertically in a cylindrical shape. When the coupling portion 114 is coupled to the port connection portion 333, the outer surface of the coupling portion 114 and the inner surface of the port connection portion 333 may be in contact with each other.

The port part 330 may include an inflow opening/closing part (not illustrates) that controls the inflow of the medicinal liquid into the port inlet 331. When the medicinal liquid in the internal space 100s of the chamber 110 flows into the connection tube 310 through the port extension 332 of the port part 330, the inflow opening/closing part closes the port inlet 331 to prevent the medicinal liquid from flowing out to the outside.

The upstream end of the connection tube 310 is connected to the port extension 332 of the port part 330 to allow the medicinal liquid in the internal space 100s of the chamber 110 to flow toward the downstream of the connection tube 310. The connection tube 310 interconnects the port part 330 and the air filter 500.

The air filter 500 is configured to filter air in the medicinal liquid and discharge the air to the outside. The air filter 500 is coupled to the downstream end of the connection tube 310. The air filter 500 is coupled to the injection-part-fixing device 700. The air filter 500 forms a filter flow path 500p. The filter flow path 500p is connected to the upstream end of a guide flow path 700p of the injection-part-fixing device 700, which will be described later, and configured to guide the flow of medicinal liquid to the guide flow path 700p. The upstream end of the filter flow path 500p is connected to the downstream end of the connection flow path 300p.

The air filter 500 may include a filter casing 510 connected to the connection tube 310 and a hydrophobic filter 520 disposed within the filter casing 510. The hydrophobic filter 520 is configured to allow air to pass therethrough, but prevent liquid from passing therethrough. An air vent (not illustrated) configured to discharge air filtered by the hydrophobic filter 520 to the outside is formed in the air filter 500.

The injection-part-fixing device 700 is configured to enable the injection part 2 to be coupled thereto. For example, the injection-part-fixing device 700 and the injection part 2 may be coupled to each other using a screw. The injection-part-fixing device 700 forms the guide flow path 700p configured to guide the flow of the medicinal liquid to the injection part 2. The upstream end of the guide flow path 700p is connected to the downstream end of the filter flow path 500p.

The injection-part-fixing device 700 includes a fixed body 720 that provides the guide flow path 700p. The injection-part-fixing device 700 includes a filter coupling portion 730 coupled to the air filter 500. The injection-part-fixing device 700 includes the injection-part-coupling portion 700 configured to be coupled to the injection part 2. The injection-part-fixing device 700 may include a support leg 750 configured to support a fixed body 720 with respect to a patient's skin. For example, the support leg 750 may be fixed to a member (not illustrated) attached to the patient's skin, allowing the injection-part-fixing device 700 to be disposed at a predetermined position and at a predetermined angle with respect to the patient's skin.

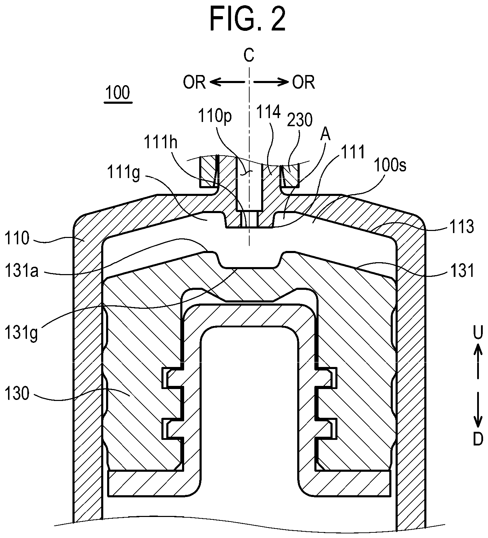

FIG. 2 is an enlarged cross-sectional view of the air-filled medicinal liquid pumping apparatus of FIG. 1. Referring to FIG. 2, the air-filled medicinal liquid pumping apparatus 100 may include a chamber 110 and a plunger 130.

The chamber 110 may form an injection flow path 110p connecting the air-filled internal space 100s with the outside of the chamber 110. The injection flow path 110p may be formed by the coupling portion 114 of the chamber 110. The injection flow path 110p is connected to the connection tube part 300. The upstream end of the injection flow path 110p is connected to the internal space 100s of the chamber 110. The upstream end of the injection flow path 110p is a connection hole 111h, which will be described later. The downstream end of the injection flow path 110p is connected to the upstream end of the connection flow path 300p. The injection flow path 110p may be connected to the connection tube 310 via the port part 330. The medicinal liquid flowing in through the port inlet 331 of the port part 330 is flowable into the internal space 100s of the chamber 110 through the injection flow path 110p. The injection flow path 110p may be located on the upper side of the chamber 110. The injection flow path 110p may be formed in a tubular shape extending in the vertical direction.

The chamber 110 may have an inner upper end surface 113 that defines the upper end of the internal space 100s. The chamber 110 may have an inner protrusion 111 protruding downward from the inner upper end surface 113. The inner protrusion 111 may protrude downward from the central portion of the inner upper end surface 113. The inner protrusion 111 may be provided with a connection hole 111h, which is one end of the injection flow path 110p. The connection hole 111h may be provided at the protruding end of the inner protrusion 111. An external medicinal liquid may flow into the internal space 100s of the chamber 110 through the connection hole 111h. The medicinal liquid in the internal space 100s may flow out to the outside through the connection hole 111h.

The chamber 110 may form an air collection groove 111g constituting a portion of the internal space 100s. The air collection groove 111g may be formed by being recessed upward along the circumference of the inner protrusion 111. The air collection groove 111g may surround the inner protrusion 111. The level of the inner upper end surface 113 forming the air collection groove 111g may decrease in an outward direction OR from the center of the inner upper end surface 113. For convenience of explanation in the present disclosure, an imaginary central axis C is defined as extending vertically through the connection hole 111h, and the outward direction OR is defined as the direction away from the central axis C. When air A and medicinal liquid L exist in the internal space 100s of the chamber 110, the air A can be trapped in the air collection groove 111g just before the plunger 130 moves upward as much as possible.

The plunger 130 may form the internal space 100s together with the chamber 110. The plunger 130 may be configured to be movable along the inner surface of the chamber 110. The plunger 130 may be configured to be movable vertically to change the volume of the internal space 100s. When the medicinal liquid flows into the internal space 100s, the plunger 130 may move downward to increase the volume of the internal space 100s. When the medicinal liquid flows out of the internal space 100s, the plunger 130 may move upward to reduce the volume of the internal space 100s.

The plunger 130 may have an upper surface 131 in which an insertion groove 131g is formed. The insertion groove 131g may be recessed downward. The insertion groove 131g may be recessed such that the inner protrusion 111 can be inserted therein. The insertion groove 131g may be formed in the central portion of the upper surface 131 of the plunger 130.

The upper surface 131 of the plunger 130 may be provided to be spaced apart from the inner upper end surface 113 of the chamber 110. The upper surface 131 of the plunger 130 and the inner upper end surface 113 of the chamber 110 may be spaced apart from each other to form the internal space 100s. The internal space 100s may be filled with air through the port part 330. The filled air may be located in the air collection groove 111g.

The inner upper end surface 113 of the chamber 110 and the plunger 130 may define the internal space 100s. The upper end of the internal space 100s may be defined by the inner upper end surface 113. The lower end of the internal space 100s may be defined by the upper surface 131 of the plunger 130. The air-filled medicinal liquid pumping apparatus may be manufactured in the state in which the internal space 100s is filled with air. When a medicinal liquid flows into the internal space 100s, air and the medicinal liquid may coexist in the internal space 100s. Some of the air filling the internal space 100s may flow out into the connection tube 310, flow into the air filter 500, and be discharged to the outside. Air may be trapped in the air collection groove 111g of the chamber 110. The air trapped in the air collection groove 111g may be used to push the medicinal liquid remaining in the connection tube, as described later.

The air filling the internal space 100s may be sterilized air. Through this, the possibility of causing health problems to a patient being injected with the medicinal liquid can be reduced.

The plunger 130 may include an insertion portion 131a configured to be engaged with the air collection groove 111g. The insertion portion 131a may be formed to protrude upward along the circumference of the insertion groove 131g. The insertion portion 131a may surround the insertion groove 131g. The level of the insertion portion 131a may decrease in the outward direction OR. The insertion portion 131a may be configured to be engaged with the air collection groove 111g when the plunger 130 moves upward. When the plunger 130 moves upward and is engaged with the air collection groove 111g, the air existing in the internal space 100s may flow out along the injection flow path 110p.

When the manufacturing process is completed in the state in which the internal space is filled with air, the upper end of the plunger 130 is preferably located lower than the lower end of the inner protrusion 111. Through this, the volume of the air collection groove 111g can be sufficiently maintained to ensure that sufficient air is trapped in the air collection groove 111g. The effect of pushing out the remaining medicinal liquid can be further improved by the air sufficiently trapped in the air collection groove 111g.

In an embodiment, the air-filled medicinal liquid pumping apparatus 100 may include a pressing operation part 150 that provides power to move the plunger 130 upward. As an example, the pressing operation part 150 may be configured to press the medicinal liquid in the chamber 110 using a volumetric expansion by gas activation. As another example, the pressing operation part 150 provides a portion that can be held by a user, so that the plunger 130 can be moved upward with the user's force.

Although not illustrated, as another example, the plunger 130 may be configured to press the medicinal liquid using the elastic force of an elastic body such as a balloon. In this case, the plunger 130 may be configured to press the medicinal liquid within the balloon.

Although the pressing operation part 150 is illustrated in FIG. 1, the air-filled medicinal liquid pumping apparatus may not include the pressing operation part. As an example, the air-filled medicinal liquid pumping apparatus may be manufactured without including the pressing operation part. In this case, it is also possible for a medicinal liquid manufacturer or a user to directly connect and use a separate pressing operation part.

A method of manufacturing the air-filled medicinal liquid pumping apparatus may include a step of forming air in the internal space 100s of the chamber 110. The inner upper end surface 113 of the chamber 110 and the upper surface 131 of the plunger 130 may be manufactured to be spaced apart from each other so that the internal space 100s of the chamber 110 is filled with air.

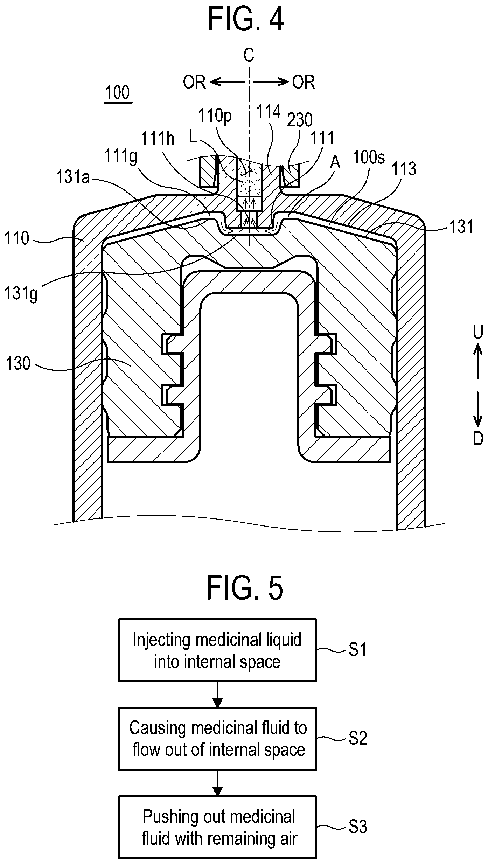

If the medicinal liquid is injected into the internal space 100s while the internal space 100s is filled with air, air will necessarily exist in the internal space 100s. When the plunger 130 rises and causes some of the medicinal liquid in the internal space 100s to flow out into the injection flow path 110p, even if some of the air in the internal space 100s flows out through the injection flow path 110p, the remaining part of the air within the internal space 100s is trapped in the air collection groove 111g and remains in the internal space 100s. When all of the medicinal liquid has flowed out from the internal space 100s, the air remaining in the air collection groove 111g may be pushed by the insertion portion 131a of the plunger 130 and flow out from the air collection groove 111g to the injection flow path 110p (see arrow in FIG. 4). The air remaining in the air collection groove 111g can push the medicinal liquid remaining in the injection flow path 110p and the connection flow path 300p. Accordingly, the amount of medicinal liquid remaining in the connection tube after using the medicinal liquid injection apparatus can be significantly reduced. The volume of the air collection groove 111g and the amount of air to be filled can be adjusted based on the volume of the flow path of the connection tube. Specific details regarding this will be described later.

The upper end of the plunger 130 may be manufactured to be located lower than the lower end of the inner protrusion 111. The upper end of the plunger 130 is located lower than the lower end of the inner protrusion 111 such that the internal space 100s is filled with sufficient air, whereby a sufficient amount of air can be induced to be trapped in the air collection groove 111g located above the lower end of the inner protrusion 111.

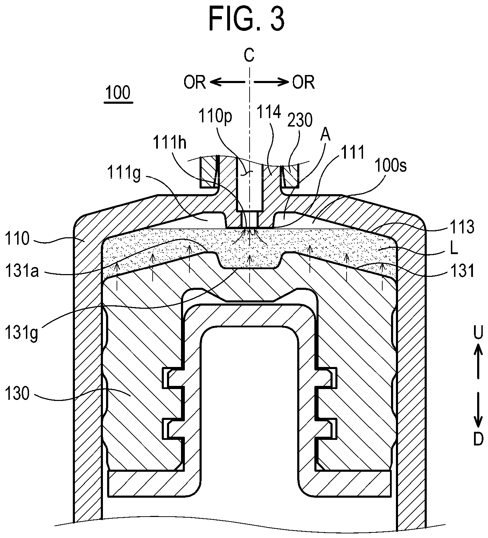

FIG. 3 is an enlarged cross-sectional view illustrating the air-filled medicinal liquid pumping apparatus. FIG. 4 is an enlarged cross-sectional view illustrating the state in which the plunger of FIG. 3 moves upward.

Hereinafter, with reference to FIGS. 1 to 5, the operating method of the air-filled medicinal liquid pumping apparatus will be described.

The operating method of the air-filled medicinal liquid pumping apparatus includes injecting a medicinal liquid into the internal space 100s (S1), causing the medicinal liquid to flow out from the internal space 100s (S2), and pushing out the medicinal liquid with residual air (S3).

In step S1, the medicinal liquid flowing in through the port part 330 may flow into the internal space 100s of the chamber 110 through the injection flow path 110p. The medicinal liquid and air may coexist in the internal space 100s of the chamber 110. When the medicinal liquid is being filled into the internal space 100s of the chamber 110, the plunger 130 moves downward, and the volume of the internal space 100s increases.

In step S2, as illustrated in FIG. 3, the plunger 130 moves upward, and the medicinal liquid in the internal space 100s flows out through the injection flow path 110p. Even if the plunger 130 moves upward, the air located in the air collection groove 111g cannot flow out to the outside through the injection flow path 110p and remains.

In step S2, when the medicinal liquid flows out to the outside, some of the air existing in the internal space 100s (i.e., the air that is not trapped in the air collection groove) may flow out into the connection flow path 300p together with the medicinal liquid. The air flowing out together with the medicinal liquid may flow into the air filter 500 through the connection tube part 300. The gas flowing into the air filter 500 may be discharged to the outside through the above-described air vent. Even if some of the air flows out from the internal space 100s, the state in which air A is trapped in the air collection groove 111g of the internal space 100s can be maintained.

In step S2, if the plunger 130 continues to move upward, all of the medicinal liquid in the internal space 100s may flow out through the injection flow path 110p. Even at this time, air remains in the air collection groove 111g of the internal space 100s.

In step S3, as illustrated in FIG. 4, the plunger 130 moves upward, and the air remaining in the internal space 100s can be discharged into the injection flow path 110p (see arrows in FIG. 4). The air flowing out to the injection flow path 110p may push out the medicinal liquid remaining in the injection flow path 110p, and may push out the medicinal liquid remaining in the injection flow path 110p and the connection flow path 300p. Accordingly, the user of the medicinal liquid pumping apparatus 100 is able to use the medicinal liquid remaining in the injection flow path 110p and the connection tube 310, thereby significantly reducing loss of the medicinal liquid.

Referring to FIGS. 1 and 3, in an embodiment, the volume of the internal space 110s located above an imaginary horizontal line that is in contact with the protruding end of the inner protrusion 111 (hereinafter referred to as "trap volume") may be larger than or equal to the sum of the volume of the injection flow path 110p and the volume of the connection flow path 300p. Here, the imaginary horizontal line means, for example, the same line as the surface line of the medicinal liquid L in FIG. 3, and the trap volume means, for example, the same volume as the volume of air trapped in the air collection groove 111g in FIG. 3. Through this, in the final step of injecting the medicinal liquid into a patient, the air corresponding to the trap volume is able to push out all of the medicinal liquid remaining in the injection flow path 110p and the connection flow path 300p, and the air that pushes out the medicinal liquid is able to be filtered by the air filter 500 before entering the patient's body.

In addition, the sum of the volume of the filter flow path 500p of the air filter 500 and the volume of the guide flow path 700p of the injection-part-fixing device 700 may be smaller than the sum of the volume of the injection flow path 110p and the volume of the connection flow path 300p. Since the air filter 500 discharges air to the outside, the medicinal liquid remaining in the medicinal liquid flow path downstream from the air filter 500 cannot be pushed out by the above-described trapped air, but the amount of medicinal liquid remaining in the flow path to be discarded may be minimized by reducing the volume of the filter flow path 500p and the volume of the guide flow path 700p.

In the foregoing, the technical idea of the present disclosure has been described with reference to some embodiments and examples illustrated in the accompanying drawings. However, it is to be understood that various substitutions, modifications, and alterations may be made without departing from the technical idea and scope of the present disclosure that can be understood by a person ordinarily skilled in the technical field to which the present disclosure pertains. In addition, such substitutions, modifications, and alterations are to be considered as falling within the scope of the appended claims.

## Claims

1. An air-filled medicinal liquid pumping apparatus comprising:
a chamber configured to form an injection flow path connecting an air-filled internal space with an outside of the chamber, wherein the chamber includes an inner upper end surface defining an upper end of the internal space, and an inner protrusion protruding downward from the inner upper end surface and including a protruding end where a connection hole, which is one end of the injection flow path, is formed; and
a plunger configured to form the internal space together with the chamber and be movable in a vertical direction along an inner surface of the chamber to change a volume of the internal space, wherein the plunger includes an upper surface forming an insertion groove recessed downward to allow the inner protrusion to be inserted in the insertion groove,
wherein the inner upper end surface and the upper surface are spaced apart from each other.

2. The medicinal liquid pumping apparatus of Claim 1, wherein an upper end of the plunger is located below a lower end of the inner protrusion.

3. The medicinal liquid pumping apparatus of Claim 1, wherein the chamber has an air collection groove recessed upward along a circumference of the inner protrusion and forming a portion of the internal space.

4. The medicinal liquid pumping apparatus of Claim 3, wherein the plunger includes an insertion portion that protrudes upward along a circumference of the insertion groove and is configured to be engaged with the air collection groove when the plunger moves upward.

5. The medicinal liquid pumping apparatus of Claim 1, wherein air filling the internal space is sterilized air.

6. The medicinal liquid pumping apparatus of Claim 1, wherein the medicinal liquid pumping apparatus is manufactured in a state in which the internal space is filled with air.

7. The medicinal liquid pumping apparatus of Claim 1 further comprising:
a pressing operation part configured to press the plunger so as to move the plunger upward.

8. A medicinal liquid injection apparatus comprising:
the air-filled medicinal liquid pumping apparatus according to Claim 1;
an injection-part-fixing device configured such that an injection part configured to be inserted into a patient's skin for injecting a medicinal liquid is coupled to the injection-part-fixing device, wherein the injection-part-fixing device is configured to form a guide flow path configured to guide a flow of the medicinal liquid to the injection part;
an air filter coupled to the injection-part-fixing device and connected to an upstream end of the guide flow path to form a filter flow path configured to guide the flow of the medicinal liquid into the guide flow path, wherein the air filter is configured to filter air in the medicinal liquid and discharge the air to the outside of the chamber; and
a connection tube part configured to connect an upstream end of the filter flow path of the air filter with a downstream end of the injection flow path of the air-filled medicinal liquid pumping apparatus, wherein the connection tube part is configured to form a connection flow path configured to guide the flow of the medicinal liquid.

9. The medicinal liquid injection apparatus of Claim 8, wherein the volume of the internal space located above an imaginary horizontal line that is in contact with the protruding end of the inner protrusion is greater than or equal to a sum of a volume of the injection flow path and a volume of the connection flow path.

10. The medicinal liquid injection apparatus of Claim 9, wherein a sum of a volume of the filter flow path and a volume of the guide flow path is smaller than the sum of the volume of the injection flow path and the volume of the connection flow path.

11. The medicinal liquid injection apparatus of Claim 8, wherein the injection-part-fixing device includes:
a fixed body configured to form the guide flow path; and
a support leg configured to support the fixed body with respect to a patient's skin.
